# EUROPÄISCHE PATENTSCHRIFT

(11) **EP 3 074 002 B1**
(45) Veröffentlichungstag und Bekanntmachung des Hinweises auf die Patenterteilung: **08.05.2019**
(21) Anmeldenummer: 14802869.9
(22) Anmeldetag: 24.11.2014
(51) Int. Cl.: A61K 31/135, A61P 13/00

(54) **TRICYCLISCHE VERBINDUNGEN ZUR BEHANDLUNG VON BELASTUNG- UND DRANGINKONTINENZ**
TRICYCLIC COMPOUNDS FOR THE TREATMENT OF STRESS AND URGE INCONTINENCE
COMPOSÉS TRICYCLIQUES POUR LE TRAITEMENT DE L'INCONTINENCE D'EFFORT ET D'URGENCE

(30) Priorität: 25.11.2013 DE 102013019618
(43) Veröffentlichungstag der Anmeldung: 05.10.2016
(73) Patentinhaber: Georges, Avlen, 61381 Friedrichsdorf (DE)
(72) Erfinder: Georges, Avlen, 61381 Friedrichsdorf (DE)
(74) Vertreter: Jacobi, Markus Alexander
(86) Internationale Anmeldenummer: PCT/EP2014/075364
(87) Internationale Veröffentlichungsnummer: WO 2015/075216

(56) Entgegenhaltungen:
- EP-A1- 0 501 705
- WO-A1-2010/102071
- US-A1- 2005 033 374
- US-A1- 2011 021 499
- WHI-AN KWON ET AL: "Effect of Low-Dose Triple Therapy Using Gabapentin, Amitriptyline, and a Nonsteroidal Anti-Inflammatory Drug for Overactive Bladder Symptoms in Patients With Bladder Pain Syndrome", INTERNATIONAL NEUROUROLOGY JOURNAL, Bd. 17, Nr. 2, 1. Januar 2013 (2013-01-01) , Seite 78, XP055166213, ISSN: 2093-4777, DOI: 10.5213/inj.2013.17.2.78
- VAN OPHOVEN ET AL: "A PROSPECTIVE, RANDOMIZED, PLACEBO CONTROLLED, DOUBLE-BLIND STUDY OF AMITRIPTYLINE FOR THE TREATMENT OF INTERSTITIAL CYSTITIS", JOURNAL OF UROLOGY, LIPPINCOTT WILLIAMS & WILKINS, BALTIMORE, MD, US, Bd. 172, Nr. 2, 1. August 2004 (2004-08-01), Seiten 533-536, XP005530879, ISSN: 0022-5347, DOI: 10.1097/01.JU.0000132388.54703.4D
- INTERSTITIAL CYSTITIS COLLABORATIVE RESEARCH NETWORK ET AL: "Effect of Amitriptyline on Symptoms in Treatment Naive Patients With Interstitial Cystitis/Painful Bladder Syndrome", JOURNAL OF UROLOGY, LIPPINCOTT WILLIAMS & WILKINS, BALTIMORE, MD, US, Bd. 183, Nr. 5, 1. Mai 2010 (2010-05-01), Seiten 1853-1858, XP027038707, ISSN: 0022-5347 [gefunden am 2010-05-01]
- OSAMU NISHIZAWA ET AL: "Guidelines for management of urinary incontinence", INTERNATIONAL JOURNAL OF UROLOGY, Bd. 15, Nr. 10, 1. Oktober 2008 (2008-10-01), Seiten 857-874, XP055166130, ISSN: 0919-8172, DOI: 10.1111/j.1442-2042.2008.02117.x
- LINK C: "MEDICAL TREATMENT OF URINARY INCONTINENCE IN WOMEN", CME BULLETIN GYNAECOLOGY, RILA PUBLICATIONS, GB, Bd. 2, Nr. 3, 1. Januar 2001 (2001-01-01), Seiten 80-83, XP001097210, ISSN: 1462-8783
- OSAMU YAMAGUCHI ET AL: "Clinical guidelines for overactive bladder", INTERNATIONAL JOURNAL OF UROLOGY, Bd. 16, Nr. 2, 1. Februar 2009 (2009-02-01), Seiten 126-142, XP055166110, ISSN: 0919-8172, DOI: 10.1111/j.1442-2042.2008.02177.x
- SANTORO G A ET AL: "Open study of low-dose amitriptyline in the treatment of patients with idiopathic fecal incontinence.", DISEASES OF THE COLON AND RECTUM DEC 2000, Bd. 43, Nr. 12, Dezember 2000 (2000-12), Seite 1676, XP008174670, ISSN: 0012-3706
- SINGH HARAMANDEEP ET AL: "Novel therapeutic usage of low-dose doxepin hydrochloride", EXPERT OPINION ON INVESTIGATIONAL DRUGS, ASHLEY PUBLICATIONS LTD., LONDON, GB, Bd. 16, Nr. 8, 1. August 2007 (2007-08-01) , Seiten 1295-1305, XP009094175, ISSN: 1354-3784, DOI: 10.1517/13543784.16.8.1295
- ELNEIL ET AL: "Complex pelvic floor failure and associated problems", BAILLIERE'S BEST PRACTICE AND RESEARCH. CLINICAL GASTROENTEROLOGY, BAILLIERE TINDALL, LONDON, US, Bd. 23, Nr. 4, 1. August 2009 (2009-08-01) , Seiten 555-573, XP026390999, ISSN: 1521-6918 [gefunden am 2009-08-06]

## Beschreibung

Die vorliegende Erfindung betrifft Zusammensetzungen für die Behandlung von Inkontinenz bei Säugetieren und Menschen unter Einsatz von tricyclischen Verbindungen. Bei der Speicherung von Urin ist bei gesunden Säugern bzw. Menschen der Blasenmuskel meist entspannt, so kann sich die Harnblase ausdehnen und füllen. Hingegen ist der Schließmuskel angespannt, so dass Urin nicht gleich wieder aus der Blase über die Harnröhre abfließen kann. Wenn die Harnblase entleert werden soll, zieht sich der Blasenmuskel zusammen. Der Schließmuskel erschlafft mit der Beckenbodenmuskulatur und der Urin kann aus der Blase durch die Harn-Röhre abfließen. Bei einer Inkontinenz ist das aufeinander abgestimmte System aus Blasenmuskel, Schließmuskel und Beckenbodenmuskulatur sowie den steuernden Nerven und Zentren in Gehirn bzw. Rückenmark nicht voll funktionsfähig. Eine derartige Störung kann z. B. durch eine Verletzung oder Erkrankung des Nervensystems verursacht sein oder organische Ursachen haben.

Für die Therapie von Harn-inkontinenz gibt es verschiedene Verfahren, die aber zum Teil aufwändig sind, zu unbefriedigenden Ergebnissen führen oder Nebenwirkungen mit sich bringen. Insbesondere fehlt es an oral verabreichbaren Verbindungen und Zusammensetzungen.

Auch sind die Behandlungskosten hoch durch teure Wirkstoffe, Windel-Verbrauch und Patienten-Compliance Probleme. Aufgabe ist es auch, durch die Bereitstellung von pharmazeutischen Produkten die Lebensqualität der Patienten zu verbessern, insbesondere deren Unsicherheit bzgl. der eigenen Inkontinenz zu beseitigen. Daher bestand ein Bedarf nach neuen Wirkstoffen und Formulierungen, die eine Behandlung bzw. Prävention von Inkontinenz, insbesondere von Belastungs- und Dranginkontinenz, bei Säugetieren und Menschen ermöglichen, ohne die o.g. Nachteile zu haben.

Zahlreiche tri- und tetracyclische Verbindungen sind im Stand der Technik als Wirkstoffe bekannt. Diese werden unter anderem in pharmazeutischen Formulierungen zur Behandlung von Depressionen eingesetzt. Die WO 2008/103442 beschreibt gepufferte Harnröhrenzäpfchen zur Behandlung einer über-aktiven Blase, wobei u. a. auch Amitriptylin, Butriptylin und Nortriptylin als anticholinerge Agentien genannt werden. US 6562813 offenbart KombinationsPräparate zur Behandlung von Inkontinenz, in denen ein erster Wirkstoff und ein Wiederaufnahme-Hemmer (Fluvoxamin, Fluoxetin und Paroxetin) kombiniert werden. WO 2005/052576 beschreibt pharmazeutische Zusammensetzungen, enthaltend Wirkstoffe, die an ein KCNK6-Polypeptid binden, wobei auch die Behandlung von Inkontinenz erwähnt wird.

Kwon et al. (Int Neurourol J., 2013, 17:78-82) lehrt eine Dreifachtherapie basierend auf der Verwendung eines Gemisches aus Amitriptylin, Gabapentin und eines nicht-steroidalen Entzündungshemmers zur Behandlung von Patienten mit Blasenschmerzen. Van Ophoven et al. (The Journal of Urology., 2004, 172:533-536) betrifft die Behandlung von interstitieller Zystitis mittel Amitriptylin. Foster et al. (The Journal of Urology, 2010, 183:1853-1858) lehrt die Wirkung von Amitriptylin auf Patienten mit interstitiellen Zystitis und Blasenschmerzen.

Nishizawa et al. (International Journal of Urolology, 2008, 15:857-874), Link (CME Bulletin Gynaecology, 2001, 2:80-83) und Yamaguchi et al. (International Journal of Urolology, 2009, 16:126-142) fassen Behandlungsmethoden zum Umgang von urinaler Inkontinenz zusammen.

EP-A 0501705 lehrt die Verwendung von Tomoxetin zur Behandlung von unteren Harnwegskrankheiten. WO 2010/102071 betrifft oral verabreichbare Dosiereinheiten zur verzögerten Freisetzung eines R-Baclofen-Prodrugs. US-A 2011/021499 betrifft Harnwegszäpfchen zur Behandlung von Inkontinenz. US-A 2005/033374 lehrt Behandlungen des Beckenbodens durch elektrische Stimulation. Santoro et al. (Diseases of the Colon & Rectum, 2000, 43:1676-1682) lehrt den Einsatz von niedrigdosiertem Amitriptylin zur Behandlung von Patienten mit idiopathischer Fäkalinkontinenz. Singh und Becker (Expert Opin. Investig. Drugs, 2007, 16:1295-1305) betrifft die therapeutische Verwendbarkeit von niedrigdosiertem Doxepinhydrochlorid bei Schlafstörungen. Elneil (Best Practice & Research Clinical Gastroenterology, 2009, 23:555-573) lehrt die Behandlung von komplexen Beckenbodenstörungen.

Gelehrt wird hierin allgemein auch der Einsatz einer tri- oder tetracyclischen Verbindung der Formel (I) 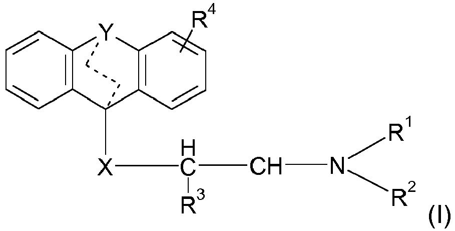 oder ein Salz (pharmazeutisch verträgliches Salz) oder ein Stereoisomer oder ein Stereoisomeren-Gemisch davon, wobei:
X für =CH- oder -CH₂-; R¹ für CH₃ oder C₂H₅; R² für CH₃ oder C₂H₅; R³ für H oder CH₃; R⁴ für H, CN oder Halogen;
wobei bei einem Tricyclus die gestrichelten Linien keine Bindungen darstellen (entfallen) und Y für -CH₂-, -CH₂-CH₂-, -CH=CH-, -CH₂-O-; -CH₂-S- oder -CH₂-NH- steht, (insbesondere für -, -CH₂-CH₂-), oder wobei bei einem Tetracyclus die gestrichelten Linien Einfachbindungen sind und (-CH₂-CH₂-) bedeuten und Y für -CH₂- oder -CH₂-CH₂- steht,
zur Verwendung für die Prävention und/oder Behandlung von Inkontinenz. Von besonderem Interesse ist dabei die Verwendung als oral anwendbare Applikation zur Prävention und/oder Behandlung von Inkontinenz.

Die vorliegende Erfindung ist gemäß den Ansprüchen definiert.

Die Erfindung betrifft eine Dosiereinheit, die gemäß den Ansprüchen charakterisiert ist, enthaltend eine Verbindung für die Prävention und/oder Behandlung von leichter bis mittelschwerer Inkontinenz wobei die Verbindung ein Tricyclus der Formel (II) ist, 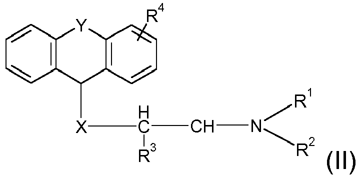 oder ein pharmazeutisch verträgliches Salz oder ein Stereoisomer oder ein Stereoisomeren-Gemisch davon,
wobei:
X für =CH- oder -CH₂-;
R¹ für CH₃ oder C₂H₅;
R² für CH₃ oder C₂H₅;
R³ für H oder CH₃;
R⁴ für H, CN oder Halogen; und
Y -C₂H₄- bedeutet.

In der Verbindung für die Prävention und/oder Behandlung von Inkontinenzkann in der Formel (I) oder (II) X die Gruppe =CH- bedeuten.

In der Verbindung für die Prävention und/oder Behandlung von Inkontinenzkann in der Formel (I) oder (II) R³ Wasserstoff bedeuten und können R¹ und R² jeweils CH₃ bedeuten.

Die Erfindung betrifft auch eine Dosiereinheit, die gemäß den Ansprüchen charakterisiert ist, enthaltend eine spezielle Verbindung für die Prävention und/oder Behandlung von Inkontinenz, insbesondere von Belastungs- und Dranginkontinenz, wobei die Verbindung der Formel (I) folgende Formel (A) hat:

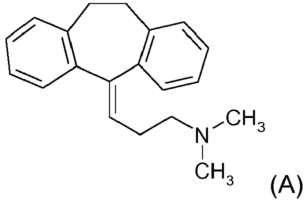

Von besonderem Interesse ist die Verbindung (A) zur oral anwendbaren Prävention und/oder Behandlung von Inkontinenz. Gelehrt wird auch eine der o.g. Verbindungen der Formeln (I), (II) oder (A) bzw. die pharmazeutisch verträglichen Salze für die Prävention und/oder Behandlung von Drang- oder Stress-Harninkontinenz.

Dabei können durch den Einsatz der Verbindungen der Formeln (I), (II) oder (A) bzw. deren pharmazeutisch verträgliche Salze, insbesondere bei oraler Applikation, Nebenwirkungen klassischer Behandlungsverfahren vermieden werden.

Offenbart wird ferner eine pharmazeutische Zusammensetzung, enthaltend eine Verbindung der Formel (I) oder (II), oder ein pharmazeutisch verträgliches Salz oder ein Stereoisomer oder ein Stereoisomeren-Gemisch davon, und mindestens einen pharmazeutisch akzeptablen Hilfsstoff für die orale Applikation zur Prävention und/oder Behandlung der Inkontinenz, insbesondere von leichter bis mittelschwerer Inkontinenz, insbesondere auch von Belastungs- und Dranginkontinenz,

Diese pharmazeutische Zusammensetzung enthält häufig eine Verbindung der Formel (A), oder ein pharmazeutisch verträgliches Salz oder ein Stereoisomer oder ein Stereoisomeren-Gemisch davon, und mindestens einen pharmazeutisch akzeptablen Hilfsstoff für die orale Applikation zur Prävention und/oder Behandlung der Inkontinenz, insbesondere von Drang- oder Stress-Harninkontinenz.

Gegenstand der Erfindung ist eine Dosiereinheit für die orale Anwendung, enthaltend eine tricyclische Verbindung der Formel (II) wie gemäß den Ansprüchen charakterisiert, oder ein pharmazeutisch verträgliches Salz oder ein Stereoisomer oder ein Stereoisomeren-Gemisch davon, insbesondere der Verbindung (A), wobei die Dosiereinheit erfindungsgemäß von 2 bis 25 mg, insbesondere 5 bis 20 mg, oftmals von 10 bis 20 mg, der Verbindung der Formel (II) wie gemäß den Ansprüchen charakterisiert oder (A) enthält. Die Dosiereinheit enthält in der Regel mindestens einen pharmazeutisch akzeptablen Hilfsstoff.

Insbesondere bei Verbindung (A) hat sich die erfindungsgemäß eingesetzte (niedrige) Dosierung von 12,5 bis 25 mg/Tag als vorteilhaft für die Behandlung bzw. Prävention der Inkontinenz erwiesen.

Diese Dosiereinheit enthält erfindungsgemäß 2 bis 25 mg, insbesondere 5 bis 20 mg, oftmals auch 12,5 mg der tricyclischen Verbindung der Formel (A), oder ein pharmazeutisch verträgliches Salz (z.B. ein Hydrochlorid) oder ein Stereoisomer oder ein Stereoisomeren-Gemisch davon:

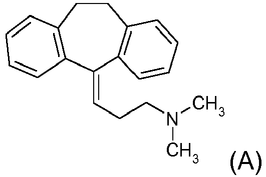

Diese Dosiereinheit enthält oftmals auch weitere Hilfsstoffe wie Lactose, Stärke, Talk, etc. Diese Dosiereinheit kann z. B. einmal täglich oral (insbesondere als Tablette) appliziert werden.

Einige der Substanzen (I) oder (II) lassen sich auch für andere medizinische Indikationen (Depressionen und chronische Schmerzen) einsetzen, jedoch oftmals in anderer (insbesondere höherer) Dosierung. Besonders Verbindung (A) ist zur Behandlung von leichter bis mittelschwerer Harninkontinenz geeignet, selbst in Dosierungen, die deutlich unterhalb des Anwendungsbereichs des bekannten tricyclischen Antidepressivums mit dem Wirkstoff Amitriptylin liegen.

Fünf Formen der Harninkontinenz werden häufig beschrieben, die unterschiedliche Ursachen haben, wobei der Stress- und Drang-Inkontinenz im Rahmen dieser Erfindung besondere Bedeutung zukommt, da hier besonders gute Behandlungs-Resultate erzielt wurden.

Bei der Belastungs- oder Stress-Inkontinenz kann der Verschluss-mechanismus zwischen Blasenhals und Harnröhre geschädigt sein, z. B. durch Unfall oder Operation.

Das Risiko einer Harninkontinenz ist z. B. bei Prostataoperationen hoch, wird die Prostata komplett entfernt, kann es zu einer Absenkung des eigentlich intakten Schließmuskels kommen. Auch ein Blasenvorfall kann zu einer Harninkontinenz führen. Auch bei Frauen kommt es oftmals zur Belastungsinkontinenz (z.B. nach der Entbindung oder durch Gebärmuttersenkung, Descensus uteri).

Bei der besonders häufig auftretenden Dranginkontinenz besteht ein Signalübertragungsfehler zwischen Blase und Gehirn, beziehungsweise Rückenmark. Schon bei geringer Blasenfüllung wird das Signal einer gefüllten Blase gegeben. Mögliche Ursachen sind können oftmals Operationen mit Nervenschäden oder -reizungen sein. Auch neurologische Erkrankungen wie Multiple Sklerose, Parkinson oder Alzheimer können eine Dranginkontinenz auslösen. Auch Hirntumor oder Schlaganfall können Ursachen sein. Auch ständige Reizungen der Blase durch Blasensteine oder Harnwegsinfekte können eine Dranginkontinenz mit sich führen. Auch Diabetes kann zur Dranginkontinenz führen.

Eine Reflexinkontinenz entsteht bei Verlust der Blasen-Schließmuskel-Koordination aufgrund von Störungen der Nerven, die die Blase steuern. Sie wird ausgelöst z. B. durch Verletzungen des Rückenmarks, Querschnittslähmung, neurologische Erkrankungen, wie Parkinson, Multiple Sklerose, Schlaganfall, Alzheimer und andere Demenzerkrankungen.

Bei einer Überlaufinkontinenz liegt ein Abflusshindernis am Blasenausgang vor, zum Beispiel durch eine vergrößerte Prostata oder eine Harnröhrenverengung.

Bei der extra-urethralen Inkontinenz besteht eine Fehlbildung des unteren Harnleiters, diese ist meist angeboren und daher langfristig zu therapieren.

Zur Behandlung der Inkontinenz-Formen Belastungs- und Dranginkontinenz können die Verbindungen der Formel (I) und insbesondere die der Formel (II) bzw. (A) vorteilhaft eingesetzt werden. Insbesondere Verbindung (A) zeigt selbst in sehr niedriger Dosierung gute nachhaltige Ergebnisse.

Dabei ist zu beachten, dass verschiedene bekannte Medikamente wie Diuretika, sonstige Antidepressiva, Neuroleptika sowie Alkohol eine bestehende Harninkontinenz sogar verstärken können.

Da unkontrollierter Harnverlust ein unangenehmes Thema, besteht ein hoher Bedarf an kostengünstigen und wirksamen Wirkstoffen, die zu einer einfachen Applikation genutzt werden können.

Oftmals sind Beckenboden-training und Elektrotherapie sowie angepasste Trinkmengen und geeignete Getränkeauswahl nicht ausreichend, insbesondere wenn Übergewicht und andere Risikofaktoren für Inkontinenz hinzukommen. Auch Injektion von Botulinustoxin ("Botox") in die Blasen-muskulatur (für Frauen und Männer bei Dranginkontinenz geeignet) sowie eine "Umspritzung" der Harnröhre mit Kollagen oder Silikon kann zwar in ausgewählten Fällen die Harnröhre stabilisieren und Symptome lindern, ist jedoch sehr aufwändig. Eine extraurethrale Inkontinenz kann der Arzt operativ behandeln, zusätzlich kann die Inkontinenz auch medikamentös behandelt werden. Mit dem bekannten Wirkstoff Duloxetin steht zwar ein Weg zur Behandlung der Belastungsinkontinenz zur Verfügung, es handelt sich jedoch um einen Serotonin-Noradrenalin-Wiederaufnahme-Hemmer, der signifikante Neben-wirkungen und hohe Therapie-Abbruchraten mit sich bringt.

Die Behandlung einer Dranginkontinenz mit Anticholinergika (z.B. Trospiumchlorid, Oxybutynin, Propiverin, Tolterodin) bewirkt, dass sich der Blasenmuskel nicht mehr zusammenzieht, was ebenfalls Nachteile hat. Bei einer Überlaufinkontinenz kann mit Alpharezeptorblocker behandelt werden, die den Blasenverschluss lockern und den Auslasswiderstand senken, wodurch die Restharnmenge vermindert wird.

Die spontane Aktivität der Harnblasenmuskulatur bei einer Reflexinkontinenz kann durch Parasympatholytika gehemmt werden. Sie bewirken, dass sich die Blase entspannt. Diese Präparate haben aber bei längerer Anwendung Nachteile.

Erfindungsgemäß ergab sich die Behandlung der Inkontinenz als besonders wirksam und mit geringen Nebenwirkungen, wenn das Antidepressivum Amitriptylin aus der Gruppe der tricyclischen Antidepressiva eingesetzt wird. Bereits eine geringe Dossierung (erfindungsgemäß 12,5 bis 25 mg) täglich, führte zur einen deutlichen Beschwerdebesserung, bei einigen Patienten auch zur Beschwerdefreiheit. Es können Dosierungen weit unterhalb der normalen Depressionstherapie (50 bis 300 mg täglich) eingesetzt werden.

Die tri- (und tetra)-cycischen Antidepressiva haben eine entspannende Wirkung auf die glatte Muskulatur der Harnblase. Dadurch kommt es zu einer Reduktion der Kontraktilität des Blasenmuskels, welche zu einer Besserung der Beschwerden führt. Das häufige Wasserlassen bei Tag und in der Nacht aus Harndrang unterbleibt, ein Urinverlust kann vermieden werden.

Als tri- und tetracyclische Verbindungen kommen die o.g. Verbindungen (I) und (II) in Frage, aber auch die Antidepressiva wie Amitriptylin und Clomipramin. Zu den trizyklischen Antidepressiva gehören auch Amitriptylinoxid, Desipramin, Dibenzepin, Doxepin, Imipramin, Lofepramin, Nortriptylin, Opipramol und Trimipramin.

Wichtige tetrazyklische Antidepressiva sind Maprotilin, Mianserin und Mirtazapin. Anwendungsgebiete dieser tri- und tetracyclischen Antidepressiva sind leichte bis sehr starke Schmerzen, Angststörungen, Schlafstörungen, Nervosität, Wechseljahresbeschwerden, Alkohol-abhängigkeit, Psychosen und Burnout.

Gelehrt werden auch ganz allgemein eine pharmazeutische Zusammensetzung (Arzneimittel), enthaltend mindestens eine Verbindung der Formel (I) oder (II), insbesondere eine Verbindung der Formel (A) sowie pharmazeutisch geeignete Hilfsstoffe. Ein Verfahren zur Herstellung der Zusammensetzung, enthaltend eine Verbindung der Formel (I) oder (II) ist ebenfalls offenbart, wobei die Verbindung der Formel (I) oder (II) mit einem pharmazeutisch geeigneten Hilfsstoff vermischt und diese Mischung in eine für die Verabreichung geeignete Form gebracht wird. Von besonderem Interesse ist dabei eine pharmazeutische Zusammensetzung (Arzneimittel), das oral anwendbar ist und zur Prävention und/oder Behandlung von Inkontinenz, insbesondere Drang- oder Stressinkontinenz, eingesetzt wird.

Beschrieben werden auch pharmazeutisch verträgliche Salze der Verbindungen der Formel (I) oder (II), insbesondere der Verbindung der Formel (A). Pharmazeutisch verträgliche Salze sind häufig aufgrund ihrer höheren Wasserlöslichkeit gegenüber den Basisverbindungen besonders für medizinische Anwendungen geeignet. Diese Salze weisen ein pharmazeutisch verträgliches Anion oder Kation auf. Geeignete pharmazeutisch verträgliche SäureAdditionssalze der erfindungsgemäß in der Dosiereinheit enthaltenen Verbindungen sind z.B. Salze anorganischer Säuren, wie Salzsäure, Bromwasserstoff-, Phosphor-, Metaphosphor-, Salpeter- und Schwefelsäure sowie organischer Säuren, wie z. B. Essigsäure, Benzolsulfon-, Benzoe-, Zitronen-, Ethansulfon-, Fumar-, Glucon-, Glykol-, Milch-, Lactobion-, Malein-, Äpfel-, Methansulfon-, Bernstein-, p-Toluolsulfon- und Weinsäure.

Geeignete pharmazeutisch verträgliche basische Salze sind insbesondere Ammoniumsalze, Alkalimetallsalze, insbesondere Natrium- und Kaliumsalze, und Erdalkalisalze, insbesondere Magnesium- und Calciumsalze, und Salze von Trometamol (2-Amino-2-hydroxymethyl-1,3-propandiol), Diethanolamin, Lysin und Ethylendiamin.

Die erfindungsgemäß in der Dosiereinheit enthaltenen Verbindungen können häufig auch in verschiedenen polymorphen Formen vorliegen, z.B. als amorphe und kristalline polymorphe Formen. Bei der vorliegenden Beschreibung beziehen sich alle Verweise auf "Verbindungen gemäß Formel (I)" auf Verbindungen der Formel (I), wie vorstehend beschrieben, sowie deren Salze und Stereoisomere.

Zur therapeutischen Anwendung der Verbindungen (I) und (II) können unterschiedliche pharmazeutische Zusammensetzungen (Zubereitungen) bereitgestellt werden. Normalerweise ist die Menge der erfindungsgemäß in der Dosiereinheit enthaltenen Verbindung, die erforderlich ist, um den gewünschten biologischen Effekt zu erreichen, abhängig von mehreren Faktoren, z. B. der gewählten Verbindung, dem zu behandelnden Individuum, der Art der Inkontinenz, und dem Alter, Geschlecht sowie klinischen Zustand des Patienten. Im Allgemeinen liegt die Tagesdosis beim Menschen im Bereich von 1 mg bis 50 mg (vorzugsweise von 2 mg bis 25 mg), erfindungsgemäß bei 12,5 mg bis 25 mg, pro Tag.

Für oral verabreichbare Einzeldosis-Formulierungen (Dosiereinheiten), wie zum Beispiel Tabletten oder Kapseln, können beispielsweise von 1 bis 50 mg, typischerweise von 2 bis 25 mg, oftmals 5 bis 20 mg, der aktiven Verbindung (I), (II) oder (A) eingesetzt werden.

Zur Therapie und Prophylaxe der oben genannten Inkontinenz-Störungen können die Verbindungen (I) und (II), insbesondere die Verbindung gemäß Formel (A), auch selbst als Verbindung verwendet werden, insbesondere für die orale Anwendung, vorzugsweise liegen sie jedoch mit mindestens einem verträglichen Hilfsstoff in Form einer pharmazeutischen Zusammensetzung bzw. Zubereitung vor. Der Hilfsstoff muss verträglich sein, d. h. er muss mit den anderen Bestandteilen der Zusammensetzung kompatibel sein.

Er darf auch nicht gesundheitsschädlich für den Patienten sein. Der Hilfsstoff kann z. B. ein Feststoff und/oder eine Flüssigkeit sein und wird vorzugsweise mit der Wirkstoff-Verbindung als Einzeldosis formuliert, beispielsweise als Tablette, die von 0,05% bis 40 Gew.-% des Wirkstoffs enthalten kann. Weitere pharmazeutisch aktive Substanzen können ebenfalls vorhanden sein, einschließlich weiterer Verbindungen der Formel (I). Die erfindungsgemäßen pharmazeutischen Zusammensetzungen, die gemäß den Ansprüchen charakterisierte Dosiereinheiten darstellen, können nach bekannten Methoden hergestellt werden. Beispielsweise werden der Wirkstoff (A) und pharmakologisch verträgliche Hilfsstoffe intensiv gemischt und dann in die gewünschte Form gebracht.

Als erfindungsgemäße pharmazeutische Zusammensetzungen, die gemäß den Ansprüchen charakterisierte Dosiereinheiten darstellen, kommen insbesondere solche in Frage, die für orale und perorale (z. B. sublinguale) Applikation geeignet sind (z.B. Filmtabletten), denkbar ist im Prinzip aber auch eine parenterale Anwendung (z.B. subkutane, intramuskuläre oder intravenöse), aber auch solche, die für eine rektale oder topische Verabreichung bevorzugt werden. Die nichtoralen Applikationen haben jedoch auch Nachteile bei der Behandlung der Inkontinenz.

Geeignete pharmazeutische Zubereitungen für die orale Verabreichung können in separaten Einheiten vorliegen, zum Beispiel als Kapseln, Lutschtabletten oder Tabletten, die jeweils eine bestimmte Menge der Verbindung gemäß Formel (I) oder (II) oder (A) enthalten. Sie können auch als Pulver oder Granulate, als Lösung oder Suspension in einer wässrigen oder nicht-wässrigen Flüssigkeit vorliegen. Auch eine Formulierung als eine Öl-in-Wasser- oder Wasser-in-ÖI-Emulsion ist möglich. Tabletten sind von besonderem Interesse, z. B. mit 12,5 oder 25 mg des Wirkstoffs.

Diese Zusammensetzungen können nach geeigneten pharmazeutischen Methoden zubereitet werden, die einen Schritt umfassen, bei dem der Wirkstoff (I) oder (II) oder (A) und der bzw. die Hilfsstoffe in Kontakt gebracht werden. Im Allgemeinen werden die Zusammensetzungen durch gleichmäßiges und homogenes Vermischen des Wirkstoffs mit dem flüssigen und/oder fein verteilten festen Hilfsmittel hergestellt, wonach das Produkt, falls erforderlich, geformt und anschließend verpackt wird. So kann beispielsweise eine Tablette hergestellt werden, indem ein Pulver oder Granulat der aktiven Verbindung verpresst oder geformt wird, gegebenenfalls mit einem oder mehreren zusätzlichen Hilfsstoffen. Gepresste Tabletten können durch Tablettieren der Verbindung in frei fließender Form, wie beispielsweise einem Pulver oder Granulat, gegebenenfalls gemischt mit einem Bindemittel, Gleitmittel, Verdünner und/oder dispergierenden Mitteln in einer geeigneten Maschine hergestellt werden. Geformte Tabletten können auch durch Formen der pulverförmigen, mit einem flüssigen Verdünnungsmittel befeuchteten aktiven Verbindung in einer geeigneten Maschine hergestellt werden.

Pharmazeutische Zusammensetzungen für die perorale Verabreichung sind z.B. Lutschtabletten, die eine Verbindung gemäß Formel (I) oder (II) oder (A) mit einem Geschmacksstoff enthalten, üblicherweise Saccharose und Gummi arabicum, und Pastillen, welche die Verbindung in einer inerten Basis wie Gelatine und Glycerin oder Saccharose und Gummi arabicum enthalten. Der Wirkstoff ist häufig in einer Konzentration von 0,1 bis 20 Gew.-% der Zusammensetzung vorhanden, insbesondere von 0,5 bis 5 Gew.-%. Bereits in niedriger Dosierung eingesetzt, kann insbesondere die Verbindung (A) bzw. deren Hydrochlorid, zu einer dauerhaften Prävention und zur Behandlung von Drang-Inkontinenz (z.B. über mehr als 6 oder 12 Monate) eingesetzt werden und den Alltag der Personen nachhaltig verbessern.

Die vorliegende Erfindung wird durch die nachfolgenden Beispiele und Ansprüche näher erläutert und ist gemäß den Ansprüchen charakterisiert:

### Beispiel 1: 42-jährige Patientin, verheiratet, 3 Kinder

Die Patientin beklagt Dauerschmerzen im Lumbalbereich, belastungsabhängige Schmerzen in der linken Schulter, depressive Störung seit circa 7 Jahren, Stressinkontinenz Grad I-II seit der Geburt des 3 Kindes vor circa 8 Jahren. Es wurde Amitriptylin (A) 12,5mg/täglich (in Form einer Filmtablette) verordnet. Nach circa 3 Wochen gab die Patientin eine Besserung der depressiven Störung an. Es wurde auch eine Besserung der Inkontinenz festgestellt.

### Beispiel 2: 52-jährige Patientin, verheiratet, 2 Kinder

Bekannte Vorerkrankungen sind: HWS/Schultersyndrom seit circa 25 Jahren, Zustand nach Gallen-OP, depressive Störung seit ca. 25 Jahren, Stress und Drang-Inkontinenz seit ca. 8 Jahren. Medikation (neben anderen): Amitriptylin 25mg oral /täglich, abends. Nach circa 2 Wochen berichtet die Patientin zunächst eine Verbesserung der Inkontinenz. Nach circa 4 Wochen mit Amitriptylin 25mg zeigte die Patientin keine Inkontinenzzeichen mehr. Nach Versuchen, das Produkt Amitriptylin abzusetzen, beklagte die Patientin wieder die Inkontinenz. Bei der Wiedereinnahme von Amitriptylin 25mg/täglich (oral) gab die Patientin deutliche Besserung der Inkontinenz an.

### Beispiel 3: 54-jähriger Patient, verheiratet, 2 Kinder

Chronische Lumboischialgie, hyperlipidämie, Depressionen seit circa 1 Jahr, Stress-und Drang-Inkontinenz Grad I-II nach Prostata-OP bei Prostatahyperplasie vor 1 Jahr. Amitriptylin 25mg, 1,0 mal abends (oral). Hier auch nach circa 2 Wochen der Einnahme von Amitriptylin zeigte der Patient auch eine deutlich Besserung der Inkontinenz Beschwerden.

## Patentansprüche

1. Dosiereinheit enthaltend 2 bis 25 mg einer tricyclischen Verbindung der Formel (II) 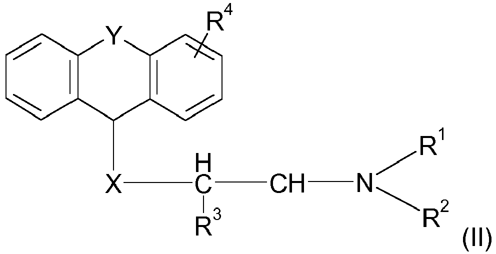 oder ein pharmazeutisch verträgliches Salz, oder ein Stereoisomer oder ein Stereoisomeren-Gemisch davon, wobei:
X für =CH- oder -CH₂-;
R¹ für CH₃ oder C₂H₅;
R² für CH₃ oder C₂H₅;
R³ für H oder CH₃;
R⁴ für H, CN oder Halogen; und
Y für -C₂H₄- steht,
und mindestens einen pharmazeutisch akzeptablen Hilfsstoff für die orale Applikation,
wobei die Dosiereinheit 0,1 bis 20 Gew.-% der Verbindung nach Formel (II) enthält,
zur Verwendung für die Prävention und/oder Behandlung von leichter bis mittelschwerer Belastungs- oder Dranginkontinenz,
wobei die oral verabreichte Tagesdosis bei 12,5 mg bis 25 mg der Verbindung der Formel (II) pro Tag liegt.

2. Dosiereinheit zur Verwendung nach Anspruch 1, wobei in der Verbindung der Formel (II) X die Gruppe =CH- bedeutet.

3. Dosiereinheit zur Verwendung nach einem der Ansprüche 1 oder 2, wobei in der Verbindung der Formel (II) oder deren pharmazeutisch verträgliches Salz R³ Wasserstoff bedeutet und R¹ und R² jeweils CH₃ bedeuten.

4. Dosiereinheit zur Verwendung nach einem der Ansprüche 1 bis 3, wobei die Verbindung der Formel (II) folgende Formel (A) hat: 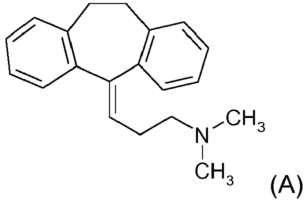 oder ein pharmazeutisch verträgliches Salz davon.

## Claims

1. Dosing unit containing 2 to 25 mg of a tricyclic compound of formula (II) 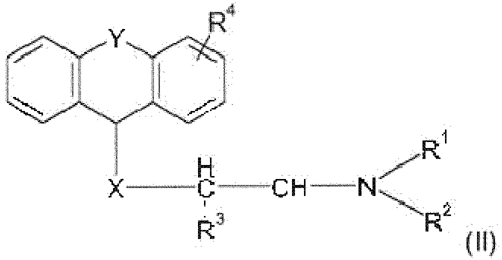 or a pharmaceutically acceptable salt, or a stereoisomer or a stereoisomeric mixture thereof, wherein:
X is =CH- or -CH₂-;
R¹ is CH₃ or C₂H₅;
R² is CH₃ or C₂H₅;
R³ is H or CH₃;
R⁴ is H, CN or halogen; and
Y is -C₂H₄-,
and at least one pharmaceutically acceptable excipient for oral application,
wherein the dosing unit contains 0.1 to 20 wt% of the compound according to formula (II),
for use for the prevention and/or treatment of mild to moderate stress or urge incontinence,
wherein the orally administered daily dose is 12.5 mg to 25 mg of the compound of formula (II) per day.

2. Dosing unit for use according to Claim 1, wherein, in the compound of formula (II), X is the group =CH-.

3. Dosing unit for use according to one of Claims 1 and 2, wherein, in the compound of formula (II) or the pharmaceutically acceptable salt thereof, R³ is hydrogen and R¹ and R² are each CH₃.

4. Dosing unit for use according to one of Claims 1 to 3, wherein the compound of formula (II) has the following formula (A): 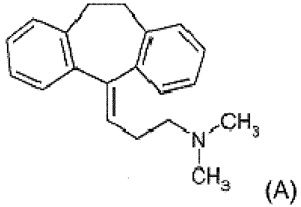 or a pharmaceutically acceptable salt thereof.

## Revendications

1. Unité de dosage contenant 2 à 25 mg d'un composé tricyclique de formule (II) 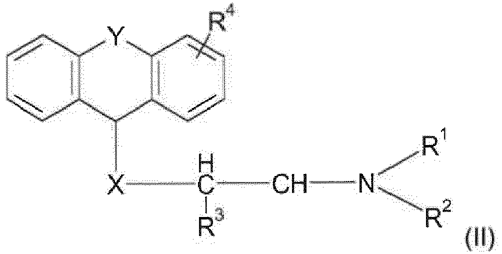 ou un sel pharmaceutiquement acceptable ou un stéréo-isomère ou un mélange de stéréo-isomères de celui-ci, dans laquelle
X représente =CH- ou -CH₂- ;
R¹ représente CH₃ ou C₂H₅ ;
R² représente CH₃ ou C₂H₅ ;
R³ représente H ou CH₃ ;
R⁴ représente H, CN ou halogène ; et
Y représente -C₂H₄-,
et au moins un adjuvant pharmaceutiquement acceptable pour l'administration par voie orale, l'unité de dosage contenant 0,1 à 20% en poids du composé selon la formule (II), pour une utilisation pour la prévention et/ou le traitement de l'incontinence d'effort ou d'urgence légère à modérée, la dose journalière administrée par voie orale se situant à 12,5 mg jusqu'à 25 mg du composé de formule (II) par jour.

2. Unité de dosage pour une utilisation selon la revendication 1, X signifiant le groupe =CH- dans le composé de formule (II).

3. Unité de dosage pour une utilisation selon l'une quelconque des revendications 1 ou 2, R³ signifiant hydrogène et R¹ et R² signifiant chacun CH₃ dans le composé de formule (II) ou dans son sel pharmaceutiquement acceptable.

4. Unité de dosage pour une utilisation selon l'une quelconque des revendications 1 à 3, le composé de formule (II) présentant la formule (A) suivante : 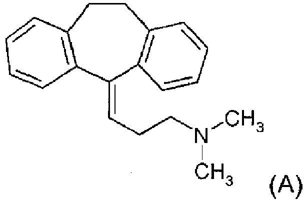 ou un sel pharmaceutiquement acceptable de celui-ci.
